(19) Europäisches Patentamt
European Patent Office
Office européen des brevets

(11) **EP 2 500 717 B1**

(12) **EUROPÄISCHE PATENTSCHRIFT**

(45) Veröffentlichungstag und Bekanntmachung des Hinweises auf die Patenterteilung:
**16.05.2018 Patentblatt 2018/20**

(21) Anmeldenummer: **12001693.6**

(22) Anmeldetag: **13.03.2012**

(51) Int Cl.:
*G01N 23/06* (2018.01)  *G01T 7/00* (2006.01)
*G01N 23/083* (2018.01)  *A61B 6/00* (2006.01)

(54) **Verfahren zum Kalibrieren zumindest einer aus mehreren Detektoren ausgebildeten Detektorzeile**

Method for calibrating at least one detector cell comprising multiple detectors

Procédé de calibrage d'au moins une cellule de détection formée de plusieurs détecteurs

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.03.2011 DE 102011014103**

(43) Veröffentlichungstag der Anmeldung:
**19.09.2012 Patentblatt 2012/38**

(73) Patentinhaber: **Fagus-GreCon Greten GmbH & Co. KG**
**31061 Alfeld (DE)**

(72) Erfinder: **Bergmann, Bernd**
**30900 Wedemark (DE)**

(74) Vertreter: **Jabbusch, Matthias et al**
**Jabbusch Siekmann & Wasiljeff**
**Patentanwälte**
**Roscherstrasse 12**
**30161 Hannover (DE)**

(56) Entgegenhaltungen:
**GB-A- 2 046 052    US-A- 6 148 057**

EP 2 500 717 B1

**Beschreibung**

Die Erfindung betrifft ein Verfahren zum Kalibrieren zumindest einer aus mehreren Detektorelementen ausgebildeten Detektorzeile, die einer hochenergetischen, sich fächerförmig ausweitenden Strahlung, welche von einer annähernd punktförmigen Quelle ausgeht, ausgesetzt ist.

Diese Strahlung tritt beim Durchdringen eines Werkstoffes mit diesem in Wechselwirkung, die es ermöglicht, physikalische Eigenschaften des Werkstoffes zu bestimmen. Verschiedene Werkstoffe werden während ihrer Herstellung bzw. während ihrer Verarbeitung mit Röntgenquellen durchstrahlt, um physikalische Eigenschaften des Werkstoffes zu bestimmen. Derartige physikalische Eigenschaften sind beispielsweise die Flächenmasse, die Dichte oder die Dicke eines beispielsweise plattenförmigen Werkstoffes, insbesondere in der Holzwerkstoffindustrie.

Für das Vermessen von Werkstoffen verwendete Detektoren sind häufig Detektorzeilen, welche aus einer Vielzahl von einzelnen Detektorelementen zusammengesetzt sind. Die einzelnen Detektorelemente haben ein voneinander unabhängiges Übertragungsverhalten. Hinzu kommen ausgehend von der punktförmigen Strahlungsquelle verschieden lange Wege für die sich fächerförmig ausweitende Strahlung auf dem Weg zu den einzelnen Detektorelementen der Detektorzeile.

Häufig hat die Detektorzeile eine größere Baulänge als der zu vermessende Werkstoff. In diesem Fall soll die Vermessung die Anforderung bestehen, dass ein gleiches Messergebnis erhalten wird, unabhängig davon, welchen konkreten Bereich der Detektorzeile der Werkstoff abdeckt.

Aufgrund der genannten Einflüsse auf die einzelnen Detektoren ist eine regelmäßige Kalibrierung der Detektoren erforderlich.

Der Erfindung liegt die Aufgabe zugrunde, ein einfaches und beschleunigt durchzuführendes Verfahren zum Kalibrieren aufzuzeigen.

Diese Aufgabe ist erfindungsgemäß durch das Verfahren im Anspruch 1 gelöst. Bei dem erfindungsgemäßen Verfahren wird eine Kalibrierung nicht mit einer Probe des Werkstoffes vorgenommen, welcher anschließend durchstrahlt werden soll. Vielmehr finden Kalibrierkörper Einsatz, deren Absorption ähnlich der Absorption des zu durchstrahlenden Werkstoffes ist. Erfindungsgemäß sind diese Kalibrierkörper aber homogen ausgebildet, damit unterscheiden sich die Kalibrierkörper beispielsweise von inhomogenen Werkstoffen wie Holz oder Zellwolle.

Für jeden Kalibrierkörper wird das Absorptionsvermögen vermessen. Die einzelnen Messwerte werden dann zur Berechnung eines Absorptionsvermögens für den Werkstoff in der Weise verwendet, dass die Absorptionsvermögen der einzelnen Kalibrierkörper einer bekannten Eigenschaft, wie z. B. der Flächenmasse, dieser Kalibrierkörper zugeordnet werden und dass das Absorptionsvermögen in Abschnitten zwischen den bekannten Eigenschaften, z. B. Flächenmassen, interpoliert wird. Mit der Messung einiger weniger Kalibrierkörper kann also ein Absorptionsvermögen für den zu vermessenden Werkstoff bestimmt werden, nach dem dann die einzelnen Detektorelemente in der Kalibrierung normiert werden.

Nach einer ersten Weiterbildung der Erfindung ist vorgesehen, dass vier bis acht Kalibrierkörper verwendet werden. Diese Anzahl von Kalibrierkörpern kann einen bestimmten Bereich einer physikalischen Eigenschaft abdecken, beispielsweise voneinander verschiedene Flächenmassen oder voneinander verschiedene Dicken. Jeder Kalibrierkörper ist homogen ausgebildet, so dass er den zu vermessenden Werkstoff als solchen abbildet. Die Kalibrierkörper sind beispielsweise aus Glas, Kunststoff oder Metall ausgebildet, so kann für das Vermessen von Holz ein Kunststoff oder ein Plexiglas eingesetzt werden.

Bei dem Kalibrieren wird nach einer Weiterbildung der Erfindung auch eine Volleinstrahlung ohne Kalibrierkörper durchgeführt. Diese Volleinstrahlung zeigt die maximale Intensität, mit der die Strahlung auf die Detektorelemente auftreffen kann. Die Vollstrahlung wird nach einer Weiterbildung auch dazu verwendet, dass für eine Interpolation des Absorptionsvermögens bzw. der Intensität zunächst eine Interpolation des Absorptionskoeffizienten $\mu$ über die Flächenmasse $f$ erfolgt. Die Intensität hat dann die Form einer e-Funktion.

So gilt für die Intensität eines Detektorelementes $d_n$:

$$i_n(f) = i_{0,n} \cdot e^{-\mu_n(f) \cdot f} \qquad n \in \left[ 0 \,|\, N \right]$$

Aus dieser Intensitätsfunktion wird eine Transformation auf eine e-Funktion mit konstantem Absorptionskoeffizienten $\mu_0$ durchgeführt. Es entsteht also eine Intensitätsfunktion, so dass für die Detektorelemente $d_n$ gilt:

$$i_n(f) = i_{0,n} \cdot e^{-\mu_n(f) \cdot f} \xrightarrow{\;T\;} i_n{'}(f) = i_{0,n} \cdot k_n \cdot e^{-\mu_0 \cdot f} = I_0 \cdot e^{-\mu_0 \cdot f} = i(f)$$

Aus der Messung homogener Kalibrierkörper $K_i$, mit konstanter Dichte und bekannter Flächenmasse $f_i$, folgt:

$$K_1, K_2, K_3, .., K_M \text{ mit } \rho_1 = \rho_2 = \rho_3 = .. = \rho_M \text{ und } f_1 < f_2 < f_{m-1} < f_m < .. < f_M$$

$$(1) \qquad i_n(f_i) = i_{0,n} e^{-\mu_n(f_i) \cdot f_i} \quad \text{mit } n \in [0 \,|\, N] \quad \wedge \quad i \in [1 \,|\, M]$$

$$\mu_n(f_i) = \mu_{n,i} \quad \textbf{diskrete Werte}$$

$$\Rightarrow \quad i_n(f_i) = i_{0,n} e^{-\mu_{n,i} \cdot f_i} = i_{n,i} \quad \Rightarrow \quad \mu_{n,i} = \frac{1}{f_i} \ln\left(\frac{i_{0,n}}{i_{n,i}}\right)$$

Beispiel für den Verlauf von (1)

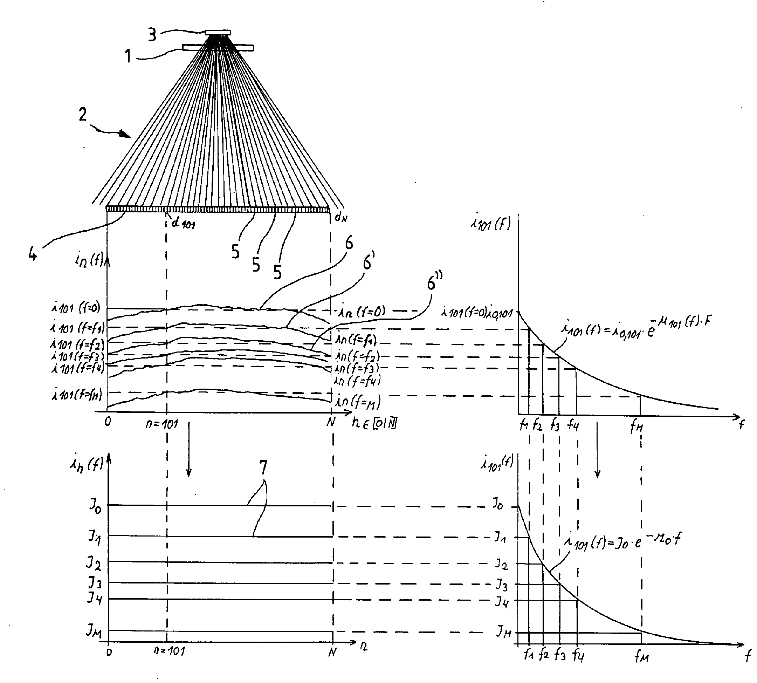

Ein Ausführungsbeispiel der Erfindung, aus dem sich weitere erfinderische Merkmale ergeben, ist in der Zeichnung dargestellt. Die einzige Figur der Zeichnung zeigt eine Seitenansicht eines Versuchsaufbaus für das erfindungsgemäße Verfahren, dem eine Grafik für die Absorption über die Elemente einer Detektorzeile und eine Grafik für eine transformierte Darstellung der Intensität der einzelnen Detektorelemente zugeordnet sind.

Bei dem erfindungsgemäßen Verfahren werden voneinander verschiedene Kalibrierkörper 1 in den Strahlengang einer punktförmigen Röntgenquelle 3 gegeben. Die Strahlen der Röntgenquelle 3 fallen in einem sich fächerförmig aufweitenden Strahlengang 2 auf eine Detektorzeile 4. Die Detektorzeile 4 ist aus einer Vielzahl von Detektoren 5 ausgebildet.

In der Zeichnung sind die Intensitätskurven 6, 6', 6" für einzelne Kalibrierkörper 1 über sämtliche Detektorelemente 5 gezeigt. Da im Strahlengang für die an den äußeren Enden der Detektorzeile 4 angeordnete Detektorelemente 5 ein längerer Strahlenweg erforderlich ist, sinkt die Intensität in diesen Bereichen ab.

Erfindungsgemäß wird mit dem gemessenen Absorptionsvermögen bzw. Intensitäten eine Transformation einer e-Funktion mit konstantem Absorptionskoeffizienten $\mu_0$ durchgeführt.

Wird $f = 0$ gesetzt, wird die $e$-Funktion = 1. Dann ergeben sich für die voneinander verschiedenen Kalibrierkörper die in der Zeichnung gezeigten horizontalen Linien 7 über die gesamte Detektorzeile 4.

Die erhaltenen normierten Werte können in eine Tabelle eingetragen werden, auf welche später die Verwender der hochenergetischen Energiequelle und der Detektorzeile 4 zurückgreifen können.

Die Zeichnung mit dem Versuchsaufbau sowie mit den vier Grafiken zeigt die Verarbeitung der Daten an einer Detektorstelle $d_{101}$. Die hier für voneinander verschiedene Probekörper 1 gewonnenen Intensitäten werden in der rechten oberen Darstellung zunächst auf den bekannten Absorptionskoeffizienten $\mu_{101}$ normiert. Die einzelnen Probekörper 1

entsprechen den Stützstellen $f_1$, $f_2$, $f_3$, die Funktion zwischen diesen Stützstellen wird interpoliert. Die rechte untere Darstellung zeigt in die Transformation dieser Intensitätsfunktion von $d_{101}$ auf einen konstanten Absorptionskoeffizienten $\mu_0$. Daraus resultiert dann die linke untere Darstellung mit normierten Intensitäten über alle Detektorelemente 5.

**Patentansprüche**

1. Verfahren zum Kalibrieren zumindest einer aus mehreren Detektoren ausgebildeten Detektorzeile, die einer von einer hochenergetischen, etwa punktförmigen Energiequelle ausgehenden, sich fächerförmig ausweitenden Strahlung ausgesetzt ist, welche einem Durchstrahlen eines Werkstoffes zum Messen physikalischer Eigenschaften aufgrund des Absorptionsvermögens des Werkstoffes dient,
**dadurch gekennzeichnet,**
**dass** wenigstens zwei jeweils homogen ausgebildete Kalibrierkörper (1) nacheinander in den Strahlengang (2) gegeben werden, deren voneinander graduell abweichende Absorption so ausgeprägt ist, dass das Absorptionsvermögen des einen Kalibrierkörpers geringer ist und des anderen Kalibrierkörpers höher ist, als das Absorptionsvermögen des zu messenden Werkstoffes, und dass für jedes Detektorelement (5) in der Detektorzeile (4) und für jeden Kalibrierkörper (1) das Absorptionsvermögen, also die Intensität der Strahlung, gemessen wird und das Absorptionsvermögen der einzelnen Kalibrierkörper (1) einer bekannten Eigenschaft dieser Kalibrierkörper (1) zugeordnet wird und dass das Absorptionsvermögen in Abschnitten zwischen diesen bekannten Eigenschaften interpoliert wird, wobei danach mit dem gemessenen Absorptionsvermögen bzw. den Intensitäten eine Transformation einer e-Funktion mit konstantem Absorptionskoeffizienten $\mu_0$ durchgeführt wird und normierte Werte über alle Detektorelemente erhalten werden.

2. Verfahren nach Anspruch 1, **dadurch gekennzeichnet, dass** vier bis acht Kalibrierkörper (1) verwendet werden.

3. Verfahren nach Anspruch 1 oder 2, **dadurch gekennzeichnet, dass** Kalibrierkörper (1) mit bekannten Flächengewichten verwendet werden.

4. Verfahren nach einem der vorhergehenden Ansprüche, **dadurch gekennzeichnet, dass** Kalibrierkörper aus Glas, Kunststoff oder Metall verwendet werden.

5. Verfahren nach einem der vorhergehenden Ansprüche, **dadurch gekennzeichnet, dass** eine Volleinstrahlung ohne einen Kalibrierkörper (1) durchgeführt wird.

6. Verfahren nach einem der vorhergehenden Ansprüche, **dadurch gekennzeichnet, dass** die Absorptionsvermögen der einzelnen Kalibrierkörper (1) den bekannten Flächengewichten dieser Kalibrierkörper zugeordnet werden und dass das Absorptionsvermögen in Flächengewichtsabschnitten zwischen diesen Flächengewichten interpoliert wird.

7. Verfahren nach Anspruch 6, **dadurch gekennzeichnet, dass** für eine Interpolation des Absorptionsvermögens bzw. der Intensität zunächst eine Interpolation der Absorptionskonstante $\mu$ auf die Flächenmasse f Volleinstrahlung erfolgt.

8. Verfahren nach Anspruch 7, **dadurch gekennzeichnet, dass** für jedes Detektorelement (5) das Absorptionsvermögen bzw. die Intensität auf eine E-Funktion mit Konstante $\mu_0$ transformiert wird.

**Claims**

1. A method for the calibration of at least one detector array formed from a plurality of detectors, which is exposed to fan-shaped radiation emanating from a high-energy, approximately punctiform energy source, which serves to irradiate a material for purposes of measuring physical properties on the basis of the absorption capacity of the material,
**characterised in that**,
at least two calibration bodies (1), in each case homogeneously formed, are introduced in succession into the beam path (2), whose absorptions, slightly differing from one another, are distinctive **in that** the absorption capacity of the one calibration body is less than, and of the other calibration body is greater than, than the absorption capacity of the material to be measured,
and **in that**,

the absorption capacity, that is to say, the intensity of the radiation, is measured for each detector element (5) in the detector array (4) and for each calibration body (1), and the absorption capacity of the individual calibration bodies (1) is associated with a known property of these calibration bodies (1),
and **in that**
the absorption capacity is interpolated in stages between these known properties, wherein
with the measured absorption capacity, that is to say, the intensities, a transformation of an e-function with a constant absorption coefficient $\mu_0$ is then performed, and normalised values are obtained over all detector elements.

2. The method in accordance with claim 1,
   **characterised in that**,
   four to eight calibration bodies (1) are used.

3. The method in accordance with claim 1 or 2,
   **characterised in that**,
   calibration bodies (1) with known masses per unit area are used.

4. The method in accordance with one of the preceding claims,
   **characterised in that**,
   calibration bodies of glass, plastic or metal are used.

5. The method in accordance with one of the preceding claims,
   **characterised in that**,
   a full irradiation is performed without a calibration body (1).

6. The method in accordance with one of the preceding claims,
   **characterised in that**,
   the absorption capacities of the individual calibration bodies (1) are associated with the known masses per unit area of these calibration bodies,
   and **in that**
   the absorption capacity is interpolated in mass per unit area stages between these masses per unit area.

7. The method in accordance with claim 6,
   **characterised in that**,
   for an interpolation of the absorption capacity, that is to say, the intensity, firstly an interpolation of the absorption constant $\mu$ over the mass per unit area f is made with full irradiation.

8. The method in accordance with claim 7,
   **characterised in that**,
   for each detector element (5), the absorption capacity, that is to say, the intensity is transformed into an E-function with constant $\mu_0$.

## Revendications

1. Procédé pour le calibrage d'au moins une rangée de détecteurs formée par plusieurs détecteurs, laquelle est exposée à un rayonnement, partant d'une source d'énergie à haute énergie approximativement ponctuelle, s'élargissant en éventail, lequel sert à une irradiation d'un matériau pour mesurer des propriétés physiques sur la base de la capacité d'absorption du matériau,
   **caractérisé en ce**
   qu'au moins deux corps de calibrage (1), respectivement réalisés de façon homogène, sont introduits successivement dans la trajectoire des rayons (2), dont l'absorption divergeant progressivement l'un de l'autre est caractéristique d'une manière telle, que la capacité d'absorption de l'un des corps de calibrage est plus faible, et celle de l'autre corps de calibrage est plus forte que la capacité d'absorption du matériau à mesurer, et
   **que** pour chaque élément de détecteur (5) dans la rangée de détecteurs (4) et pour chaque corps de calibrage (1), la capacité d'absorption, à savoir l'intensité du rayonnement, est mesurée et la capacité d'absorption des corps de calibrage (1) individuels est attribuée à une propriété connue de ces corps de calibrage (1), et
   **que** la capacité d'absorption est interpolée par sections entre ces propriétés connues, dans lequel on réalise ensuite, avec la capacité d'absorption mesuré ou les intensités, une transformée d'une fonction exponentielle avec un coefficient d'absorption constant $\mu_0$, et des valeurs normalisées sont obtenues sur tous les éléments de détecteur.

**2.** Procédé selon la revendication 1, **caractérisé en ce que** quatre à huit corps de calibrage (1) sont utilisés.

**3.** Procédé selon la revendication 1 ou 2, **caractérisé en ce que** des corps de calibrage (1) avec des poids au mètre carré connus sont utilisés.

**4.** Procédé selon l'une des revendications précédentes, **caractérisé en ce que** des corps de calibrage en verre, plastique ou métal sont utilisés.

**5.** Procédé selon l'une des revendications précédentes, **caractérisé en ce qu'**une irradiation complète est réalisée sans corps de calibrage (1).

**6.** Procédé selon l'une des revendications précédentes, **caractérisé en ce que** les capacités d'absorption des corps de calibrage (1) individuels sont attribuées aux poids au mètre carré connus de ces corps de calibrage et que la capacité d'absorption est interpolée par sections de poids au mètre carré entre ces poids au mètre carré.

**7.** Procédé selon la revendication 6, **caractérisé en ce que**, pour une interpolation de la capacité d'absorption ou de l'intensité, il y a tout d'abord une interpolation de la constante d'absorption $\mu$ sur la masse au mètre carré f d'irradiation complète.

**8.** Procédé selon la revendication 7, **caractérisé en ce que**, pour chaque élément de détecteur (5), la capacité d'absorption ou l'intensité est transformée en une fonction exponentielle avec une constante $\mu_0$.

$i_{101}(f=0)$

$i_{101}(f=f_1)$

$i_{101}(f=f_2)$

$i_{101}(f=f_3)$

$i_{101}(f=f_4)$

$i_{101}(f=f_M)$

$i_n(f)$

$i_n(f=0)$

$i_n(f=f_1)$

$i_n(f=f_2)$

$i_n(f=f_3)$

$i_n(f=f_4)$

$i_n(f=f_M)$

$0 \quad n=101 \qquad N \quad h \in [0|N]$

$i_{101}(f)$

$i_{101}(f=0) i_{0,101}$

$$i_{101}(f) = i_{0,101} \cdot e^{-\mu_{101}(f) \cdot F}$$

$f_1 \; f_2 \; f_3 \; f_4 \qquad f_M \qquad f$

$i_h(f)$

$J_0$

$J_1$

$J_2$

$J_3$

$J_4$

$J_M$

$0 \quad n=101 \qquad N \quad n$

$i_{101}(f)$

$$i_{101}(f) = J_0 \cdot e^{-\mu_0 \cdot f}$$

$J_0$

$J_1$

$J_2$

$J_3$

$J_4$

$J_M$

$f_1 \; f_2 \; f_3 \; f_4 \qquad f_M \qquad f$

$d_{101}$

$d_N$

1, 2, 3, 4, 5, 5, 5, 6, 6', 6'', 7